# EUROPÄISCHE PATENTSCHRIFT

(11) **EP 3 331 472 B1**
(45) Veröffentlichungstag und Bekanntmachung des Hinweises auf die Patenterteilung: **06.03.2019**
(21) Anmeldenummer: 16754228.1
(22) Anmeldetag: 03.08.2016
(51) Int. Cl.: A61F 2/44

(54) **IMPLANTAT ZUM VERSCHLIESSEN EINES DEFEKTS IM ANULUS FIBROSUS EINER BANDSCHEIBE**
IMPLANT FOR SEALING A DEFECT IN THE ANULUS FIBROSUS OF AN INTERVERTEBRAL DISC
IMPLANT POUR L'OBTURATION D'UN DÉFAUT DANS L'ANNEAU FIBREUX D'UN DISQUE INTERVERTÉBRAL

(30) Priorität: 04.08.2015 DE 102015112799
(43) Veröffentlichungstag der Anmeldung: 13.06.2018
(73) Patentinhaber: Richter, Marcus, 65191 Wiesbaden (DE)
(72) Erfinder: Richter, Marcus, 65191 Wiesbaden (DE)
(74) Vertreter: WSL Patentanwälte Partnerschaft mbB
(86) Internationale Anmeldenummer: PCT/EP2016/068579
(87) Internationale Veröffentlichungsnummer: WO 2017/021467

(56) Entgegenhaltungen:
- WO-A2-2007/078978
- US-A1- 2004 111 161
- US-A1- 2007 067 040
- US-A1- 2007 100 354

## Beschreibung

### Technischer Hintergrund

Die vorliegende Erfindung betrifft ein Implantat zum Verschließen eines Defekts im Anulus fibrosus einer Bandscheibe mit einem Halteabschnitt zum Einführen in den Defekt, einem mit einem inneren Ende des Halteabschnitts verbundenen Abdichtelement und einem mit dem äußeren Ende des Halteabschnitts verbundenen Befestigungselement, das an einem an die Bandscheibe angrenzenden Wirbelkörper fixierbar ist, um den Halteabschnitt in einer festen Position zu halten, wobei das flexible Abdichtelement in einer Draufsicht von vorn entlang der Längsachse des Halteabschnittes in einem ersten Zustand eine reduzierte Projektionsfläche hat und in einem zweiten Zustand eine erweiterte Projektionsfläche hat, die mindestens das Doppelte der reduzierte Projektionsfläche beträgt.

Bandscheiben bestehen aus zwei Teilen. Einem äußeren Anulus fibrosus (Faserring) und einem inneren Nucleus pulposus (Gallertkern). Der Anulus fibrosus besteht aus konzentrischen Schichten von kollagenen Bindegewebsfasern (Außenzone), die nach innen allmählich in Faserknorpel (Innenzone) übergehen und den Nucleus pulposus umgeben. Der Nucleus pulposus ist ein zellarmes gallertartiges Gewebe mit einem hohen Wassergehalt. Er wirkt wie ein Wasserkissen stoßdämpfend und nimmt Stöße bei einer Bewegung der Wirbelsäule auf. Die zwischen zwei Wirbelkörpern angeordnete Bandscheibe begrenzt den Abstand zwischen den Endplatten dieser Wirbelkörper und unterstützt als elastisch deformierbares Dämpfungselement auch die Beweglichkeit der Wirbelsäule. Die unterhalb und oberhalb der Bandscheibe angrenzenden Deckplatten bzw. Endplatten der benachbarten Wirbelkörper begrenzen das sogenannte Bandscheibenfach.

Im Rahmen der mit zunehmendem Lebensalter unvermeidlichen Abnutzung der Bandscheibe kommt es häufig zu Defekten in Form eines Aufreißens oder Aufplatzens des Anulus fibrosus. Durch einen solchen Defekt können Teile des Nucleus pulposus aus der Bandscheibe austreten, was üblicherweise als Bandscheibenvorfall bezeichnet wird.

Der durch den Defekt austretende Nucleus pulposus kann, insbesondere wenn er nach dorsal in Richtung des Spinalkanals der Wirbelsäule austritt, auf neurale Strukturen drücken. Führt dies zu neurologischen Ausfällen und/oder Schmerzen, kann eine Operation notwendig werden. Bei einer solchen Operation werden die ausgetretenen Teile des Nucleus pulposus entfernt. Der Defekt im Anulus fibrosus wird dadurch jedoch noch nicht verschlossen. Konkret weist der Anulus fibrosus ein Loch bzw. eine mehr oder weniger breite Unterbrechung auf, wobei weiterhin Teile des gallertartigen Bandscheibenkerns austreten und erneut zu Beschwerden führen können. Im Rahmen einer ersten Notbehandlung oder für ein besseres Einbringen des Halteabschnitts und des Abdichtelementes kann der Defekt auch chirurgisch erweitert und/oder präpariert werden.

Um einen Defekt im Anulus fibrosus einer Bandscheibe dauerhaft abzudichten und ein Austreten des Nucleus pulposus zu verhindern, werden u.a. die eingangs definierten Implantate verwendet. Das bekannte Implantat zum Verschließen eines Defekts im Anulus fibrosus einer Bandscheibe, wie es z. B. in der WO 2007/078978 A2 beschrieben ist, weist einen Halteabschnitt mit einem am angrenzenden Wirbelkörper fixierbaren Knochenanker auf. Der Halteabschnitt wird mit seinem inneren Ende und dem daran befestigten Abdichtelement voran in den Defekt eingeführt. Die reduzierte Projektionsfläche des Abdichtelements auf eine Fläche senkrecht zu der Längsachse des Halteabschnitts erlaubt das Hindurchführen durch den Defekt entlang dieser Längsachse. Das äußere Ende des Halteabschnitts schließt dann mehr oder weniger bündig mit der Außenseite des Anulus fibrosus ab. Das Abdichtelement hat die Form eines am inneren Ende des Halteabschnitts angebrachten Lappens, der nach dem Einbringen durch den Anulus fibrosus entfaltet werden und danach den gesamten freien Defektquerschnitt von der Innenseite des Anulus fibrosus her abdichten soll. Der Knochenanker wird in das Bandscheibenfach eingeführt und ist damit in der Deck- bzw. Endplatte eines angrenzenden Wirbelkörpers verankert, um damit das Implantat an dem Wirbelkörper zu fixieren.

Der an die Innenwand des Anulus fibrosus anzulegende Lappen des Implantates gemäß WO 2007/078978 A2 muss in einem ersten Zustand zusammengefaltet sein und in diesem ersten Zustand vor und/oder neben dem Halteabschnitt liegen. In der Draufsicht von vorn entlang der Längsachse des Halteabschnitts hat das Abdichtelement somit in dem ersten Zustand eine kleine Querschnittsfläche (= Projektionsfläche auf eine Ebene senkrecht zu Längsachse des Halteabschnitts) und ist in diesem Zustand durch die den Defekt bildende Öffnung in dem Anulus Fibrosus in das Innere der Bandscheibe einführbar. In einem zweiten Zustand ist das Abdichtelement entfaltet und liegt an der Innenseite des Anulus Fibrosus an, wobei es den Defekt vollständig abdecken und abdichten soll. In diesem Zustand ist die Querschnitts- bzw. Projektionsfläche des Abdichtelements, gesehen entlang der Achse des Halteabschnitts typischerweise mindestens doppelt so groß wie in dem ersten Zustand.

Die erwähnte Längsachse des Halteabschnitts ist in Wesentlichen definiert durch die Einschubrichtung diese Abschnitts in eine Öffnung, die wiederum bestimmt ist durch die Geometrie des Halteabschnitts und der daran angeordneten Elemente. Die Längsachse kann man auch definieren als Verbindung des äußeren und inneren Endes des Haltabschnitts.

Das Implantat gemäß WO 2007/078978 A2 hat vor allem den Nachteil, dass der theoretisch an die Innenwand des Anulus fribrosus anlegbare Lappen sich nur schwer oder gar nicht in die gewünschte Lage ausbreiten lässt und dass der Druck im Inneren der Bandscheibe diesen Lappen verschiebt und durch den Defekt wieder nach außen drückt.

Weiterhin verliert die Bandscheibe durch ein Austreten des Nucleus pulposus, aber auch alterungsbedingt allmählich an Volumen und es kommt zu einer Druckreduktion in der Bandscheibe. Die Höhe der Bandscheibe nimmt ab und damit auch die Höhe des Bandscheibenfachs. Der in dem Bandscheibenfach angeordnete Knochenanker der WO 2007/078978 A2 kann daher bei einer nahezu unvermeidbaren Degeneration der Bandscheibe die angrenzenden Endplatten beschädigen und zusätzliche Bewegungseinschränkungen verursachen. Der im Bandscheibenfach dauerhaft verbleibende Knochenanker kann außerdem eine interkorporelle Fusion, beispielsweise mit "Cages" (ALIF, TLIF, PLIF, XLIF), zu einem späteren Zeitpunkt erschweren.

Die US 2004/0111161 A1 offenbart ein künstliches Implantat aus einem nicht fließfähigen Polymermaterial zum Behandeln eines Bandscheibenvorfalls, wobei das künstliche Implantat als Ersatz für den natürlichen Nucleus pulposus in das Bandscheibenfach eingeführt wird. Weiterhin sind Befestigungsmittel vorgesehen, die das Implantat gegen ein Herauswandern aus dem Bandscheibenfach durch einen Defekt im Anulus fibrosus sichert. Die Befestigungsmittel umfassen ein Halteteil mit einem Verankerungsende und einem Blockierungsende, wobei das Verankerungsende an einem Wirbelkörper befestigt wird und das Blockierungsende so angeordnet ist, dass es das künstliche Implantat an dem Austritt aus dem Bandscheibenfach hindert, mit dem künstlichen Implantat aber nicht verbunden ist. Eine flüssigkeitsdichte Abdichtung des Defekts im Anulus fibrosus erfolgt durch die Vorrichtung nicht und ist auch nicht erforderlich, da das künstliche Implantat nicht fließfähig ist.

Die US 2007/0067040 A1 beschreibt eine Vorrichtung zur Reparatur eines Defekts im Anulus fibrosus, welche im Wesentlichen kreuzförmig ausgebildet ist und horizontale Abschnitte und vertikale Abschnitte aufweist sowie einen Mittelabschnitt, der sich zwischen den horizontalen und vertikalen Abschnitten befindet und über dem Defekt im Anulus fibrosus angeordnet wird. Die vertikalen Abschnitte werden in den über dem Bandscheibenfach angeordneten Wirbelkörpern verankert, und die horizontalen Abschnitte werden mit Klammern am Anulus fibrosus befestigt. Weiterhin ist optional ein Pfropfen aus einem wachstumsfördernden Material vorgesehen, der locker in der Öffnung im Anulus fibrosus angeordnet wird und den Durchtritt von Fluiden, Zellen und anderem Material zulassen soll, um die Heilung der Bandscheibe zu fördern. Das wachstumsfördernde Material kann beispielsweise ein allogenes Gewebe sein. Das Anbringen der Vorrichtung ist äußerst aufwändig im chirurgischen Eingriff und kann einen Defekt am Anulus fibrosus nicht dauerhaft abdichten.

Die US 200710100354 A1 beschreibt ein Reparatursystem für einen Defekt im Anulus fibrosus, wobei ein flächiges Abdichtmittel in einem zusammengefalteten Zustand durch den Defekt in das Bandscheibenfach eingebracht wird, anschließend darin entfaltet wird und sich über den Defekt und die daran angrenzenden Innenwände des Anulus fibrosus legt. Das Abdichtmittel wird anschließend mit Befestigungsmitteln an der Wand des Anulus fibrosus befestigt. Auch diese Art der Abdichtung eines Defekts im Anulus fibrosus hat sich als nicht dauerhaft dicht gegen den Austritt von Nucleus pulposus erwiesen.

### Aufgabe der Erfindung

Vor diesem Hintergrund ist es Aufgabe der vorliegenden Erfindung, ein Implantat zum Verschließen eines Defekts im Anulus fibrosus einer Bandscheibe bereitzustellen, welches, einen sicheren Sitz in dem Defekt gewährleistet und ein Austreten des Nucleus pulposus sicher verhindert.

### Beschreibung der Erfindung

Diese Aufgabe wird für ein eingangs beschriebenes Implantat zum Verschließen eines Defekts im Anulus fibrosus einer Bandscheibe mindestens teilweise dadurch gelöst, dass am äußeren Ende des Halteabschnitts ein zum Halteabschnitt abgewinkelter Befestigungsschenkel angeordnet ist, der für die Befestigung an einer knöchernen Struktur eines zu der Bandscheibe benachbarten Wirbelkörpers ausgelegt ist und der mit Hilfe eines gegenüber dem Befestigungsschenkel beweglichen Fixierelements an dem Wirbelkörper befestigbar ist, wobei das flexible Abdichtelement mindestens teilweise elastisch verformbar und in Richtung des entfalteten Zustands vorgespannt ist und/oder interne Verstärkungen zum aktiven Entfalten des Abdichtelementes aufweist.

Soweit sich aus dem Zusammenhang nicht anderes ergibt, werden im Rahmen der vorliegenden Beschreibung die Begriffe "Außen" und "Innen" anschaulich so verwendet, dass eine "Innenseite" des Implantats immer die bei oder nach dem Einsetzen des Implantats dem Zentrum der Bandscheibe oder eines angrenzenden Wirbelkörpers zugewandte Seite ist, während die "Außenseite" die von der Bandscheibe bzw. dem Wirbelkörper abgewandte Seite ist.

Zum Einsetzen des Implantates wird der Halteabschnitt mit seinem inneren Ende voran in den (gegebenenfalls mit sauberen Rändern präparierten) Defekt des Anulus fibrosus eingeführt, bis der an dem äußeren Ende des Halteabschnitts abgewinkelt angeordnete Befestigungsschenkel an einem angrenzenden Wirbelkörpers anliegt, vorzugsweise an dessen Außenseite. Das am inneren (vorderen) Ende des Halteabschnitts vorgesehene flexible Abdichtelement wird dann auf der Innenseite des Anulus fibrosus quer zu Längsrichtung des Halteabschnitts in seinen zweiten Zustand mit vergrößerter Querschnittsfläche gebracht, um den Querschnitt des Defektes abzudecken.

Das Erweitern der Querschnittsfläche geschieht entweder selbsttätig, indem das flexible Abdichtelement zunächst zusammengefaltet ist, so dass es an dem vorderen Ende des Halteabschnitts durch den Defekt hindurch in das Innere der Bandscheiben einführbar ist, und das elastisch unter Vorspannung stehende Abdichtelement sich entfaltet, sobald es freigegeben wird, oder es wird durch interne Verstärkungselemente und mit Hilfe äußerer Betätigungselemente, welche mit den Verstärkungen des Abdichtelementes verbunden sind, aktiv entfaltet. In beiden Fällen wird ein hinreichend formstabiler entfalteter Zustand des Abdichtelementes erreicht, der ein Herausdrücken des Abdichtelementes oder von Teilen desselben durch die Defektöffnung verhindert. Ein Charakteristikum der vorliegenden Erfindung ist somit, dass das Abdichtelement derart ausgestaltet ist, dass es einen zusammengefalteten Zustand, in welchem es durch den Defekt in das Innere einer Bandscheibe einführbar ist, und einen entfalteten oder aufgespannten Zustand einnehmen kann, der stabil ist. Die Mittel hierfür sind ein ausreichend steifes aber dennoch elastisch verformbares Material und/oder entsprechende Verstärkungen und Versteifungen des Abdichtelementes.

Als an dem vorderen bzw. inneren Ende des Halteabschnitts, das von dem Befestigungsschenkel weg weist, angebrachtes Abdichtelement ist vorzugsweise ein aufspannbares Lecksegel angeordnet, welches durch den Defekt in das Innere der Bandscheibe hineingeführt wird und sich nach dem Entfalten bzw. Aufspannen an die Innenseite des Anulus fibrosus anlegen kann.

In seinem aufgespannten Zustand steht das Lecksegel in einer Draufsicht auf die vordere Stirnseite des Halteabschnitts mindestens auf zwei gegenüberliegenden Seiten und vorzugsweise auch noch in einer dritten Richtung (nach oben oder unten) über die Querschnittsfläche des Halteabschnitts über und dichtet den Defekt bzw. die innere Öffnung des Defektes in dem Anulus fibrosus ab. Das Lecksegel ist dabei so bemessen, dass es typischerweise in allen Richtungen und insbesondere in der Ebene der Bandscheibe und vorzugsweise auch in einer hierzu senkrechten Richtung deutlich über die Ränder eines typischen Defektes hinausragt und auf der Innenseite des Anulus fibrosus um den Defekt herum großflächig anliegt. Anders ausgedrückt beträgt bei einer Draufsicht auf das vordere Ende des Halteabschnitts die Projektionsfläche des Abdichtelementes (auf die Ebene senkrecht zur Blickrichtung) mindestens das Doppelte der reduzierten Projektionsfläche, die das Abdichtelement während des Einbringens durch den Defekt aufweist.

Das Lecksegel kann zum schnellen und besseren Einheilen mit einem das Zellwachstum fördernden Material beschichtet oder daraus hergestellt sein. Der Halteabschnitt und auch der Befestigungsschenkel können aus einem vom Körper resorbierbaren Material hergestellt sein, die Ihre Halte- und Fixierfunktion für das Abdichtelement solange behalten, bis das Abdichtelement fest mit der Bandscheibe verwachsen ist.

Das Implantat und dessen Lecksegel können bei Bedarf in verschiedenen Größen und Ausgestaltungen bereitgestellt werden, wobei aber die Abmessungen und Formen zweckmäßigerweise so gewählt werden, dass sie jeweils einen großen Bereich von Anwendungsfällen abdecken.

Der sich abgewinkelt zu dem Halteabschnitt erstreckende Befestigungsschenkel, der mit seiner Innenseite an einem angrenzenden Wirbelkörper anliegt, wird vor oder nach dem Aufspannen des Lecksegels mit Hilfe des Verbindungsmittels an dem Wirbelkörper befestigt.

Die Länge des Halteabschnitts liegt typischerweise zwischen etwa 3 und 8 mm, der Befestigungsschenkel hat typischerweise eine Länge zwischen etwa 5 und 15 mm, wobei die verwendeten Maße von der Anatomie und Größe der jeweiligen Patienten abhängen und demzufolge dem Chirurgen sinnvollerweise ein entsprechender Satz von Implantaten unterschiedlicher Größe zur Verfügung gestellt wird. Dabei kann es auch zweckmäßig sein, wenn Befestigungsschenkel und Halteabschnitt lösbar und eventuell auch gelenkig miteinander verbunden sind, so dass man unterschiedliche Befestigungsschenkel und Halteabschnitte miteinander kombinieren kann. Es versteht sich, dass eine lösbare Gelenkverbindung zwischen Befestigungsschenkel und Halteabschnitt so gesichert ist, dass diese Verbindung sich bei einem eingesetzten Implantat nicht selbsttätig lösen kann.

Ein gemäß einer Ausführungsform vorgesehenes Lecksegel ist zum Einbringen in den Innenraum einer Bandscheibe durch einen Defekt in dem Anulus fibrosus zunächst geschlossen bzw. zusammengefaltet und wird erst, nachdem es in den Innenraum der Bandscheibe eingebracht wurde, aufgespannt. Ein Lecksegel ist ein flächiger, flexibler Gegenstand, wie zum Beispiel eine Membran, ein Tuch oder ein Lappen. Der Bandscheibeninnendruck hilft, das einmal aufgespannte Lecksegel vom Inneren der Bandscheibe her gegen den Anulus fibrosus zu drücken. Die internen bzw. in das Lecksegel integrierten Verstärkungen stellen sicher, dass das Lecksegel nicht durch den in der Bandscheibe herrschenden Druck in sich zusammengeschoben und durch die Defektöffnung herausgedrückt wird. Das aufgespannte Lecksegel deckt die innere Öffnung des Defektes im Anulus fibrosus vollständig ab und liegt flächig auf der Innenseite des Anulus fibrosus auf. Aufgrund der Fixierung durch Befestigungsschenkel und Halteabschnitt und wegen der inneren Verstärkungen bzw. Versteifungen des Lecksegels behält das Lecksegel dauerhaft seine Position bei und kann so innerhalb relativ kurzer Zeit fest mit dem Anulus fibrosus verwachsen. Danach werden Halteabschnitt und Befestigungsschenkel nicht mehr benötigt. Sie könnten deshalb, ebenso wäre auch das Fixierelement ohne weiteres aus einem vom Körper eines Patienten langsam resorbierbaren Material bestehen. Entsprechende, geeignete Materialien sind beispielsweise Polyactid oder Eisen- Tricalciumphosphat.

In (nicht beanspruchten) Ausführungsform ist das Lecksegel aus PTFE hergestellt. Die inneren Verstärkungen des Lecksegels, die mit Körpergewebe (dem Bandscheibenmaterial) nicht in Kontakt kommen, können aus einem elastischen und stabilen metallischen Material bestehen z. B. aus Federstahl oder einer Titandrahtlegierung.

In einer (nicht beanspruchten) Ausführungsform der Erfindung weist wenigstens die Seite des Lecksegels, die im aufgespannten Zustand des Lecksegels dem Anulus fibrosus zugewandt ist, bioaktive Substanzen auf, wie beispielsweise Wachstumsfaktoren, Zelladhäsionsvermittler und antibakterielle Beschichtungen, wie sie beispielsweise auch in anderen Bereichen der Knochenchirurgie Verwendung finden. Hierzu kann die Oberfläche des Lecksegel bewusst aufgeraut oder porös sein. Die bioaktiven Substanzen bewirken, dass das Lecksegel mit dem Anulus fibrosus schneller und leichter verwächst oder einheilt und so eine feste Verbindung mit dem Anulus fibrosus eingeht.

Das Lecksegel weist in einer (nicht beanspruchten) Ausführungsform mehrere Segmente in Form von Lappen oder Ausbauchungen auf, die miteinander verbunden sind und/oder sich einem aufgespannten Zustand des Lecksegels abschnittsweise überlappen. Ein einstückiges Lecksegel kann mehrere Lappen oder Ausbauchungen aufweisen und einzelne Lappen oder Segmente des Lecksegels können auch durch Falz-, Falt- und/oder Nahtlinien voneinander abgrenzt sein. In einer Ausführungsform sind mehrere Segmente zu einem Lecksegel zusammengesetzt, wobei benachbarte Segmente miteinander verklebt, vernäht, verschweißt und/oder verwoben sind. Die Form und Erstreckung der einzelnen Lappen des Lecksegels richtet sich nach den anatomischen Gegebenheiten. Auf jeden Fall soll das Lecksegel mindestens teilweise über den Querschnitt des Halteabschnitts und insbesondere auch über den Querschnitt eines typischen Defekts im Anulus fibrosus überstehen, wobei diese Querschnitte zumindest in der bevorzugten Ausführungsform nahezu übereinstimmen. Hierzu können Halteabschnitt mit verschiedenen (typischen) Querschnitten bereitgestellt werden.
In einer Ausführungsform umfasst das Lecksegel zwei Segmente, die sich in einer Draufsicht auf die Innenseite des Halteabschnitts in einem aufgespannten Zustand des Lecksegels auf gegenüberliegenden Seiten über den Querschnitt des Halteabschnitts hinaus erstrecken und den Defekt in der Bandscheibe vorzugsweise in deren gesamter Höhe bzw. Dicke überrücken.

In einer anderen Ausführungsform weist das Lecksegel mindestens drei oder vier sich vorzugsweise sternförmig von dem Halteabschnitt erstreckende Segmente auf, wobei benachbarte Segmente vorzugsweise miteinander verbunden sind und/oder sich in einem aufgespannten Zustand des Lecksegels überlappen.
In einer weiteren Ausführungsform sind einige Segmente oder alle Segmente des Lecksegels blattförmig ausgestaltet. Beispiele sind in den Figuren dargestellt.
Metallische und/oder nichtmetallische Verstärkungen im Inneren des Lecksegels, sind vorzugsweise elastisch vorspannbar und stellen eine Kraft zum Aufspannen des Lecksegels bereitstellen. Das so vorgespannte, zusammengefaltete Lecksegel kann sich nach dem Einführen in die Bandscheibe selbsttätig öffnen oder ein Aufspannen durch zusätzliche Betätigungsmittel, wie sie nachfolgend noch beschrieben werden, unterstützen. Beispielsweise können die Verstärkungen in das Material des Lecksegels eingearbeitete oder mit dem Lecksegel verbundene Metalldrähte und/oder verstärkte Fasern sein.

In einer (nicht beanspruchten) Ausführungsform ist durch den Halteabschnitt von seinem äußeren zu seinem inneren Ende hin ein Betätigungsmittel, vorzugsweise ein Zugmittel, hindurch geführt, das mit dem Lecksegel oder dessen Segmenten derart verbunden ist, dass mit Hilfe des Betätigungsmittels das Lecksegel aufgespannt werden kann. Durch aufspannen des Lecksegels kann der Defekt im Anulus fibrosus abgedichtet werden.
In einer Ausführungsform weist das Lecksegel einen Schirmmechanismus auf. Bei einem Schirmmechanismus weisen das Lecksegel bzw. einige oder alle Segmente eine vorzugsweise rippenartige Verstärkung auf, die mit einer oder mehreren Streben verbunden ist, sowie eine Gelenkverbindung mit einer Zugstange, die mit dem Lecksegel verbunden ist.

In einer weiteren (nicht beanspruchten) Ausführungsform ist ein Draht oder ein Zugfaden vorgesehen, der mit dem Lecksegel oder dessen Segmenten verbunden ist, so dass durch Ziehen an dem Draht oder Faden entweder das Lecksegel aktiv entfaltet oder ein vorgespanntes Lecksegel freigegeben wird, um sich selbsttätig zu entfalten.
Gemäß einer Ausführungsform ist das Befestigungselement hier als am äußeren Ende des Halteabschnitts angeordneter Befestigungsschenkel ausgebildet, der im Gegensatz zu dem oben diskutierten Stand der Technik nicht in das Bandscheibenfach eingeführt wird, sondern sich derart abgewinkelt vom äußeren Ende des Halteabschnitts erstreckt, dass er außen an einem Wirbelkörper anliegen kann.

In einer Ausführungsform der Erfindung sind der Befestigungsschenkel und der Halteabschnitt in einem festen Winkel zueinander einstückig ausgebildet. In einer alternativen Ausführungsform kann der Winkel zwischen Halteabschnitt und Befestigungsschenkel stufenweise oder stufenlos variabel sein und insbesondere kann der Befestigungsschenkel gelenkig mit dem äußeren Ende des Halteabschnitts verbunden sein. Im implantierten Zustand beträgt die Abwinkelung des Befestigungsschenkels gegenüber dem Halteabschnitt typischerweise zwischen 60 und 110 Grad, wobei der Winkel zwischen der Einschubrichtung des Halteabschnitts von seinem äußeren zu seinem inneren Ende hin und der Richtung zu messen ist, die durch die Verbindung des Befestigungsschenkels am äußeren Ende des Halteabschnitts mit dem freien Ende des Befestigungsschenkels definiert ist..

Der Halteabschnitt kann beispielsweise eine quaderförmige, zylindrische, stabförmige oder ellipsoide Grundform haben und ist gegenüber dem Befestigungsschenkel abgewinkelt. Die Länge des Halteabschnitts zwischen dem Befestigungsschenkel und dem Ansatz des Abdichtelementes am inneren Ende des Halteabschnitts entspricht dabei mindestens der typischen Dicke eines Anulus fibrosus (z. B. etwa 5 mm). In einer Ausführungsform ist der Halteabschnitt einstückig mit dem Befestigungsschenkel ausgeführt.

Der Halteabschnitt kann gegebenenfalls auch ein elastisch oder teilweise auch plastisch verformbarer Körper, z. B. ein Hohlkörper sein, um sich bei weiterer Degeneration der Bandscheibe einer geringer werdenden Höhe des Bandscheibenfaches anpassen zu können.

Damit das Implantat bei seiner bestimmungsgemäßen Verwendung durch die Fixierung an einer Umfangsfläche des benachbarten Wirbelkörpers in seiner Position und Orientierung relativ zu der Bandscheibe gehalten wird, ist der Befestigungsschenkel dafür ausgelegt, an eine knöcherne Struktur eines an die abzudichtende Bandscheibe angrenzenden Wirbelkörpers seitlich angelegt und mittels eines separaten Fixierelementes daran befestigt zu werden.

Dabei weist gemäß einer Ausführungsform der Befestigungsschenkel im Abstand zu dem Halteabschnitt eine Querbohrung für das Hindurchführen eines im Wirbelkörper zu verankerndes Fixierelement auf.

Das Fixierelement stellt die feste Verbindung des Befestigungsschenkels mit der knöchernen Struktur des angrenzenden Wirbelkörpers her. Auf ein in das Bandscheibenfach eingeführtes Fixierelement oder Knochenanker kann auf diese Weise verzichtet und eine weitere Beschädigung der Endplatte des Wirbelkörpers bei einer fortschreitenden Degeneration der Bandscheibe vermieden werden.

Im einfachsten Fall besteht das Fixierelement aus einem Nagel oder einer Schraube, die, gegebenenfalls mit Hilfe eines Spreizdübels, in dem Wirbelkörper befestigt werden und sich durch die passende Querbohrung in dem seitlich bzw. an der Umfangsfläche des Wirbelkörpers anliegenden Befestigungsschenkel hindurch erstreckt, um den Befestigungsschenkel so an dem Wirbelkörper zu fixieren

Das Fixierelement ist getrennt von dem Befestigungsschenkel beweglich (zum Beispiel drehbar. Kann jedoch in einer Ausführungsform unverlierbar an dem Befestigungsschenkel angebracht sein. Das Fixierelement kann darüber hinaus eine Sicherung gegen das Lösen von einer Knochenstruktur aufweisen, wozu im Falle einer Schraube oder eines Dübels zum Beispiel eine Verdrehsicherung gehören kann.

In einer Ausführungsform kann die Querbohrung zur Längsrichtung des Halteabschnitts - unabhängig von der Abwinkelung des Befestigungsschenkels zum Halteabschnitt - unter einem Winkel im Bereich von 0° bis 30° geneigt sein, wobei die Richtung dieser Neigung abhängig von dem genauen Ort des Defektes und den jeweiligen anatomischen Verhältnissen unterschiedlich sein kann. Eine gegenüber dem Befestigungsschenkel geneigte Querbohrung kann die Handhabbarkeit des Implantats vereinfachen, wenn der Befestigungsschenkel aufgrund anatomischer Gegebenheiten unter einem bestimmten Winkel relativ zum Befestigungsschenkel besser erreichbar ist da durch den Neigungswinkel der Querbohrung auch ein entsprechendes Fixierelement bzw. eine Befestigungsschraube geneigt verläuft und entsprechende Instrumente zur Herstellung der Verbindung des Implantats mit dem Wirbelkörper unter demselben Neigungswinkel in den Körper des Patienten eingeführt werden können, um so die ansonsten unvermeidbare Verletzung von Dornfortsätzen oder sonstigen Skelett- oder Weichteilen zu reduzieren oder vollständig zu verhindern.

Der Befestigungsschenkel kann beispielsweise aus einem kurzen Steg, einer Platte oder einer Leiste bestehen, die sich quer zu der Längsachse des Halteabschnitts erstreckt. Der Halteabschnitt und das Befestigungselement können gemeinsam eine L-Form haben, wobei der eine Schenkel der L-Form an einen Wirbelkörper anlegbar und der den zweiten Schenkel bildende Halteabschnitt in den Defekt des Anulus fibrosus einführbar ist. Es versteht sich, dass die Größe und Form des Befestigungsschenkels derart gewählt sind, dass der Halteabschnitt im Wesentlichen vollständig in den Anulus fibrosus eingeführt werden kann, während der die Querbohrung aufweisende Bereich des Befestigungsschenkels gleichzeitig an die Umfangsfläche eines an die Bandscheibe angrenzenden Wirbelkörpers seitlich anlegbar ist.

Die Begriffe des sich "quer" zu dem Halteabschnitt erstreckenden Befestigungsschenkels, oder eine "L-Form" bildenden Implantats umfassen hier nicht nur Ausführungsformen, bei welchen sich der Halteabschnitt und der Befestigungsschenkel von einem gemeinsamen Verbindungsbereich aus exakt senkrecht zueinander erstrecken, sondern auch solche, bei denen die beiden L-Schenkel (Halteabschnitt und Befestigungsschenkel) irgendeinen Winkel zwischen 60° und 110° miteinander einschließen.

In einer Ausführungsform ist eine Adapterhülse vorgesehen, die vorzugsweise in unterschiedlichen Ausrichtungen in der Querbohrung aufgenommen werden kann und die ihrerseits eine Bohrung aufweist, deren Achse (nach dem Einsetzen der Adapterhülse) zu der Längsachse der Querbohrung unter einem Winkel im Bereich von 0° bis 30° geneigt ist. Mit Hilfe einer solchen Adapterhülse ist es möglich, die Neigungsrichtung der Bohrung zur Aufnahme des Fixierelementes relativ zum Befestigungsschenkel und zum Halteabschnitt noch nachträglich zu variieren. Für die sichere Kraftübertragung vom Fixierelement auf den Befestigungsschenkel kann die Querbohrung im Befestigungsschenkel beispielsweise als Stufenbohrung und die Außenfläche der Adapterhülse entsprechend abgestuft ausgebildet sein und es können darüber hinaus Mittel vorgesehen sein, die Adapterhülse in verschiedenen Orientierungen bezüglich der Achse der Querbohrung zu fixieren.

Gegebenenfalls kann die Adapterhülse in der Querbohrung in einer gewünschten Ausrichtung fest vormontierbar sein.

In noch einer weiteren Ausführungsform weist der Halteabschnitt eine sich in Längsrichtung von außen nach innen durch denselben und gegebenenfalls auch durch einen Abschnitt des Befestigungsschenkels hindurch erstreckende Bohrung auf, die durch ein Septum oder einen Pfropfen aus elastischem Material verschlossen ist und als Applikationsport dient. Durch einen solchen Pfropfen können mit Hilfe einer Injektionsnadel, die durch das elastische Material des Pfropfens hindurch gestoßen wird, zu therapeutischen und/oder diagnostischen Zwecken Fluide in das Innere der Bandscheibe eingebracht oder aus dem Inneren der Bandscheibe entnommen werden, ohne den Anulus fibrosus weiter zu beschädigen oder gar das Implantat wieder entfernen zu müssen. Das elastische Material stellt sicher, dass sich der durch die Injektionsnadel erzeugte Kanal in dem Septum oder Pfropfen beim Zurückziehen der Nadel sofort wieder dicht verschließt. Vorzugsweise erstreckt sich die Bohrung geradlinig durch den Halteabschnitt und, soweit erforderlich, durch den Befestigungsschenkel. Das Material des Applikationsports ist vorzugsweise so auszuwählen, dass nach dem Durchbohren und wieder Zurückziehen einer geeigneten Hohlnadel sich der beim Durchbohren geschaffene Zufuhrkanal in dem Material wieder vollständig und sicher verschließt. In einer Ausführungsform ist der Applikationsport aus Silikon hergestellt.

Bei einem Defekt des Anulus fibrosus tritt üblicherweise ein Teil des für den Innendruck und die dämpfende Funktion der Bandscheibe verantwortlichen Nucleus pulposus aus und kann auch während der operativen Beseitigung des Defekts, beispielsweise vor oder während des Einsetzens des erfindungsgemäßen Implantats, nicht ohne weiteres aufgefüllt werden. Der Verschluss des Defekts muss zunächst einmal ausheilen bzw. die dichtenden Elemente des Implantats müssen mit dem Anulus fibrosus verwachsen, um bei den hohen Drücken und den damit verbundenen Kräften, die in der Bandscheibe wirken, eine ausreichende Abdichtung gegen ein erneutes Austreten von Nucleus pulposus oder eines Ersatzmittels zu gewährleisten. Andererseits wäre es vorteilhaft, die von Nucleus pulposus zumindest teilweise entleerte Bandscheibe erneut zu befüllen, damit die Bandscheibe ihre dämpfende Funktion wieder weitestgehend vollständig erfüllen kann. Hier kann mit Vorteil der Applikationsport in dem erfindungsgemäßen Implantat genutzt werden, um den Anulus fibrosus nach dem Verschließen des Defekts und dem ausreichenden Ausheilen bzw. Verwachsen des Verschlusses, was durchaus erst Wochen oder Monate nach dem operativen Eingriff zum Verschließen des Defekts der Fall sein kann, mit einem Ersatzmittel für Nucleus pulposus zu befüllen. Das Ersatzmittel kann jedes beliebige für solche Zwecke geeignete Mittel sein, beispielsweise körpereigene Zellkulturen oder auch ein anderes Mittel zur Regeneration des Nucleus pulposus. Das Ersatzmittel wird mittels einer durch den Applikationsport zu stoßenden Injektionsnadel in das Bandscheibenfach eingebracht. Neben oder anstelle von Ersatzmitteln können selbstverständlich auch andere Stoffe eingebracht werden, beispielsweise pharmazeutisch wirksame Stoffe, wie Antibiotika, die auch unmittelbar nach dem operativen Einsetzen des Implantats eingebracht werden können, um eine postoperative Infektion zu verhindern.

Der Halteabschnitt und/oder der Befestigungsschenkel des Implantats oder Teile hiervon, können aus einem resorbierbaren Material, vorzugsweise Polyactid oder Eisen-Tricalciumphosphat, bestehen. Resorbierbare Materialien ermöglichen eine Verwendung des Implantats selbst bei geringen Höhen des Bandscheibenfachs und vermeiden eine Beschädigung der angrenzenden Wirbelkörperendplatten.

### Beschreibung von Ausführunqsbeispielen

Weitere Vorteile, Merkmale und Anwendungsmöglichkeiten der vorliegenden Erfindung werden deutlich anhand der folgenden Beschreibung bevorzugter Ausführungsformen und der dazugehörigen Figuren. Es zeigen:
- Figur 1:: eine schematische, perspektivische Ansicht auf ein Implantat gemäß einer Ausführungsform der vorliegenden Erfindung;
- Figur 2:: einen Seitenansicht des erfindungsgemäßen Implantates nach Figur 1;
- Figur 3:: verschiedene Varianten der Anordnung von Halteabschnitten und Befestigungsschenkel.
- Figur 4: zwei Varianten von Befestigungsschenkeln
- Figur 5: einen Längsschnitt durch eine Bandscheibe mit einem erfindungsgemäßen Implantat nach Figur 1;
- Figur 6:: eine Draufsicht auf die Innenseite eines Implantats gemäß einer Ausführungsform der vorliegenden Erfindung mit einem aufgespannten Lecksegel;
- Figur 6a: Einen überhöhten Querschnitt A-A durch das Lecksegel in Figur 6.
- Figur 7a,b: die Anordnung und Lage des Implantates nach dem Einbringen in eine Bandscheibe.
- Figur 8a -c:: Seitenansichten eines Implantats gemäß der vorliegenden Erfindung mit einem von außen betätigbaren Entfaltungsmechanismus für ein Lecksegel in verschiedenen Entfaltungszuständen.
- Figur 9a-c: Ein Implantat mit einem Befestigungsschenkel zur verdrehsicheren Aufnahme von Fixierelementen.

Alle Figuren sind im Hinblick auf Maße und Maßverhältnisse und die genauen Umrissformen nur schematischer Natur. Insbesondere scharfe Kanten und Übergänge in den dargestellten Ausführungsformen können in der Realität abweichend geformt und z. B. abgerundet sein.

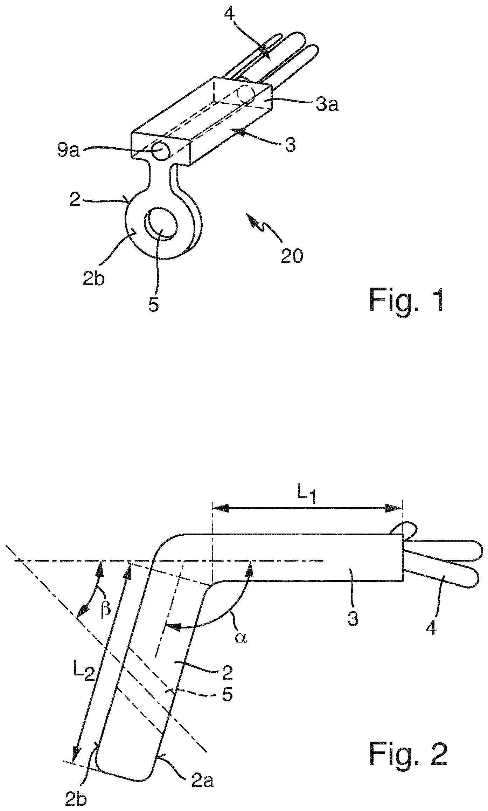

In Figur 1 ist eine perspektivische Ansicht eines Implantats 1 gemäß einer Ausführungsform der Erfindung dargestellt. Das Implantat 1 weist einen in einer Seitenansicht L-förmigen Körper 20 mit einem Befestigungsschenkel 2 auf, der eine durchgehende Bohrung 5 sowie eine (in Figur 1 nicht sichtbare) Innenseite 2a und gegenüber liegend eine Außenseite 2b hat. Der andere Schenkel des L-förmigen Körpers 20 bildet den Halteabschnitt 3, der zum Einführen in einen Defekt in dem Anulus fibrosus einer Bandscheibe ausgestaltet ist. An dem inneren Ende 3a des Halteabschnitts 3 ist ein aufspannbares Lecksegel 4 angeordnet, das in Figur 1 in seinem geschlossenen, zusammengefalteten Zustand angedeutet ist. Der Halteabschnitt 3 kann eine zentrale Längsbohrung 9a zur Aufnahme eines hier nicht dargestellten Stopfens aufweisen.

In einem von dem Halteabschnitt 3 beabstandeten Bereich des Befestigungsschenkels 2, der dafür ausgelegt ist, mit seiner Innenseite 2a an eine knöcherne Struktur eines zu der abzudichtenden Bandscheibe benachbarten Wirbelkörpers seitlich, genauer gesagt an dessen Umfangsfläche, angelegt zu werden, erstreckt sich die Bohrung 5 zur Aufnahme eines Fixierelementes 6 (in Figur 1 nicht dargestellt), das sich durch den Befestigungsschenkel 2 von der Außenseite 2b zur Innenseite 2a und im implantierten Zustand in einen angrenzenden Wirbelkörper hinein erstreckt.

Figur 2 zeigt ein Implantat in einer Seitenansicht, anhand welcher verschiedene Kenngrößen und Maße des Implantates erläutert werden können. Der Winkel zwischen der Längserstreckung des Halteabschnitts und der Längserstreckung des Befestigungsschenkels 2 ist mit α bezeichnet und kann zwischen 70 und 130 Grad betragen. Der Winkel β zwischen der Achse der Bohrung 5 und der Längserstreckung des Halteabschnitts 2 ist unabhängig von dem Winkel α und kann z. B. Werte zwischen 0 und 60 Grad annehmen.

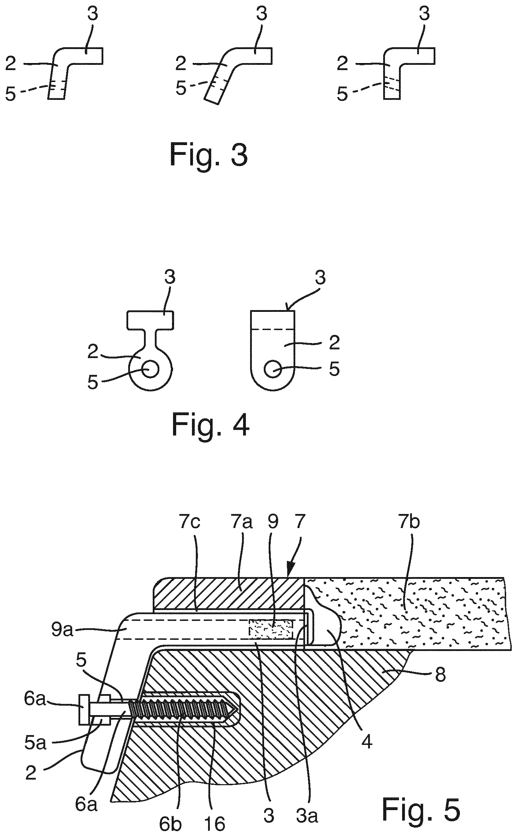

Die Figuren 3 und 4 zeigen seitliche und rückwärtige (von der Außenseite 2b her) Ansichten verschiedener Variationen von Halteelementen 3 und Befestigungsschenkeln 2. Wie man sieht, kann der Winkel α zwischen Befestigungsschenkel 2 und Halteabschnitt 3 variieren und auch der Winkel β der Achse der Bohrung 5 relativ zum Befestigungsschenkel 2 oder zum Halteabschnitt 3 kann variieren und verläuft nicht zwingend parallel zu der Längsrichtung des Halteabschnitts 3.

Figur 4 ist eine Draufsicht von außen entlang der Längsachse des Halteabschnitts, die im Wesentlichen der Einschubrichtung des Halteabschnitts in den Defekt 7c entspricht. Man sieht, dass der Befestigungsschenkel 2 unterschiedliche Formen annehmen kann, wobei es im Wesentlichen auf seine Positioniermöglichkeit an einem Wirbelkörper 8 und die Fixierung an diesem ankommt, was hier vorzugsweise mit Hilfe einer Bohrung 5 und sich durch diese hindurch erstreckende Fixierelemente 6 realisiert wird.

Die Länge L1 des Halteabschnitts 3 liegt typisch zwischen 3 und 10 mm und auch die Länge L2 des Befestigungsschenkels 2 liegt in dieser Größenordnung oder etwas darüber. Die Größe des Befestigungsschenkels 2 ist insbesondere so bemessen, dass er in einem Abstand zur Endplatte des an die Bandscheibe angrenzenden Wirbelkörpers Befestigungselemente aufweist, in diesem Fall in Form der Bohrung 5 und beispielsweis einer Schraube 6 mit einem Dübel 16, wie in Figur 5 dargestellt.

Figur 5 zeigt eine ähnliche Ausführungsform in einem schematisch dargestellten, in eine Bandscheibe 7 eingesetzten Zustand in einem Querschnitt durch die Bandscheibe 7 senkrecht zu deren Ebene im Bereich eines Defekts 7c.

Die angedeutete Bandscheibe 7 weist in ihrem Anulus fibrosus 7a einen Defekt 7c auf, durch den der Nucleus pulposus 7b austreten kann. Zum Verschließen des Defekts 7c ist der Halteabschnitt 3 in den Defekt 7c eingeführt. Der Halteabschnitt 3 und auch das zusammengefaltete Lecksegel 4 haben auf jeden Fall einen kleineren Querschnitt als der Defekt 7c, damit der Halteabschnitt 3 mit dem zusammengefalteten Lecksegel 4 voran in den Defekt 7c eingeführt werden kann. An dem inneren Ende 3a des Halteabschnitts 3 ist das Lecksegel 4 in seinem entfalteten bzw. aufgespannten Zustand dargestellt. In dem aufgespannten Zustand schmiegt sich das Lecksegel 4 von innen gegen den Anulus fibrosus und dichtet so den Defekt 7c in dem Anulus fibrosus 7a ab. Der in der gallertartigen Masse des nucleus pulposus 7b herrschende Bandscheibeninnendruck drückt das Lecksegel 4 vom innen gegen den Anulus fibrosus 7a.

Das Lecksegel 4 erstreckt sich in seinem aufgespannten Zustand und in einer Draufsicht auf das innere Ende des Halteabschnitts 3 deutlich über die Querschnittsfläche des Halteabschnitts 3 und auch weit über den Querschnitt des Defektes 7c hinaus. Auf diese Weise kann das Lecksegel 4 großflächig mit der Bandscheibe verwachsen und einheilen und hat somit auch einen guten Halt in der Bandscheibe 7. Der Halteabschnitt 3 und auch der Befestigungsschenkel 2 werden danach nicht mehr benötigt und können daher auch aus einem vom menschlichen Körper allmählich resorbierbaren Material, wie zum Beispiel aus Polyactid oder Eisen-Tricalciumphosphat, bestehen.

Wie in der Ansicht gemäß Figur 5 erkennbar, weist der zum seitlichen Anlegen an eine knöcherne Struktur eines benachbarten Wirbelkörpers 8 ausgestaltete Bereich des Befestigungsschenkels 2 eine Bohrung bzw. Durchbrechung 5 zur Aufnahme einer Schraube 6 auf. Die Bohrung 5 erstreckt sich von der Außenseite 2b des Befestigungsschenkels 2 zur Innenseite 2a und hat auf der Außenseite noch eine stufenartige Erweiterung 5a zur Aufnahme eines Schraubenkopfes 6a. Das Fixierelement ist also hier eine Schraube, die einen Kopfabschnitt 6a und einen Gewindeabschnitt 6b aufweist. Der Kopfabschnitt 6a wird in der stufenartigen Erweiterung 5a aufgenommen, während der Gewindeabschnitt 6b des Fixierelements in den Knochen des Wirbelkörpers 8, konkret in einen Spreizdübel 9 in einer in den Wirbelkörper 8 eingebrachten Bohrung, eingeschraubt ist.

Durch diese seitliche Befestigung des Implantats 1 mit dem Fixierelement 6 wird unter anderem vermieden, dass ein Befestigungselement in das Bandscheibenfach eingeführt werden muss, was die Endplatten 8a benachbarter Wirbelkörper 8 in unerwünschter Weise schädigen könnte. Die Position und Orientierung des Halteabschnitts 3 ist durch die Fixierung des Befestigungsschenkels 2 an dem Wirbelkörper 8 festgelegt. Die Höhe des Halteabschnitts 3 (zwischen gegenüber liegenden Endplatten benachbarter Wirbelkörper 8 gemessen, ist auf jeden Fall deutlich kleiner als die Höhe der Bandscheibe, die den Abstand zwischen den Endplatten bestimmt. Dadurch wird vermieden, dass nach einer etwaigen, weiteren Degeneration der Bandscheibe 7 das Implantat bzw. der Halteabschnitt 3 beidseitig mit den Endplatten der Wirbelköper 8 in Kontakt kommt und deren Beweglichkeit weiter begrenzt und/oder Schmerzen verursacht.

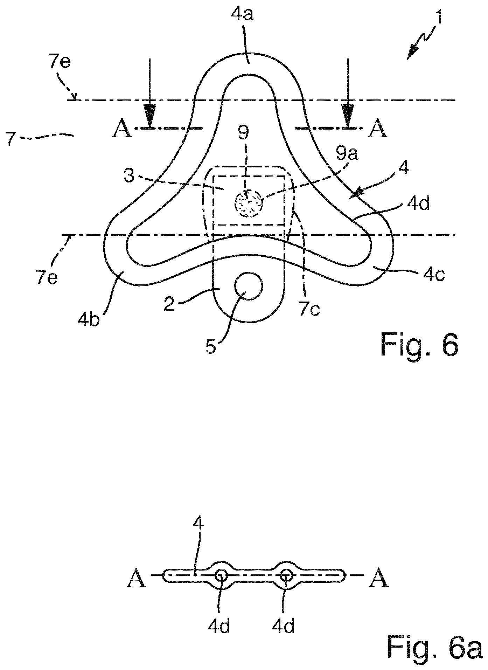

In der Figur 6 ist eine Ausführungsform eines Implantats 1 der vorliegenden Erfindung in einer Draufsicht auf die Stirnseite des Halteabschnitts 3 mit einem frei aufgespannten Lecksegel 4 gezeigt. Im frei aufgespannten Zustand würde das Lecksegel sich über die durch gestrichelte Linien 7e angedeuteten oberen und unteren Begrenzungsebenen der Bandscheibe 7 hinaus erstecken. Die Ausläufer der Segmente 4a, 4b und 4c sind deshalb nach dem Einsetzen so umgebogen, dass sie innerhalb der durch die Linien 7e definierten Begrenzungen liegen. Selbstverständlich kann das Lecksegel von vorherein auch kleinere äußere Abmessungen aufweisen, die im frei aufgespannten Zustand innerhalb der erwarteten Grenzlinien liegen oder zumindest einen geringeren Überstand aufweisen als hier dargestellt. Das Lecksegel 4 steht damit in der Draufsicht auf die Stirnseite des Halteabschnitts 3 über die Querschnittsfläche des Halteabschnitts 3 und vor allem auch über den Querschnitt eines Defektes 7c über. Das Lecksegel 4 ist einstückig aus PTFE gefertigt.

Für ein selbsttätiges Aufspannen des Lecksegels 4 sind vorgespannte Verstärkungen 4d, z. B. aus Federstahl, in das Lecksegel 4 eingearbeitet, wie aus dem in der Figur 6a gezeigten überhöhten Querschnitt durch das Lecksegel 4 entlang der Linie A-A ersichtlich ist. Beispielsweise könnte die Verstärkung 4d aus einem kreisringförmigen Draht bestehen, der zwangsweise in die in Figur 4 dargestellte Form gebracht und dadurch unter Vorspannung in das Lecksegel 4 integriert ist, wobei das Lecksegel 4 zum Einbringen durch Defekt zusätzlich zusammengefaltet wird und sich nach dem Durchtritt durch den Defekt zu der dargestellten Form aufspannt.

Wie in Figur 6 ebenfalls angedeutet ist, weist der Befestigungsschenkel 2 einen Applikationsport 9 auf, der als ein perforierbares, elastisches Septum oder ein Pfropfen in einer sich längs durch den Halteabschnitt 3 erstreckenden Bohrung 9a ausgebildet ist. Die Bohrung 9a ist durch den Pfropfen 9 verschlossen, sodass eine spitze Hohlnadel den Pfropfen 9 durchstoßen kann und anschließend durch die Nadel ein fließfähiger Stoff in das Innere der Bandscheibe eingebracht oder aus dieser entnommen werden kann. Nach dem Zurückziehen der Hohlnadel verschließt sich der durch die Hohlnadel erzeugte Durchstoßkanal aufgrund der Elastizität und inneren Adhäsion des Pfropfenmaterials selbsttätig und vollständig, wobei man vorzugsweise eine Hohlnadel verwendet, die möglichst kein Material aus dem Pfropfen herausstanzt, sondern nur das Pfropfenmaterial verdrängt. Auf diese Weise können beispielsweise Medikamente oder therapeutische Flüssigkeiten in das Innere der Bandscheibe eingebracht werden ohne den Anulus fibrosus weiter zu schädigen. Auch für diagnostische Zwecke kann durch den sich selbst verschließenden Pfropfen eine Flüssigkeitsprobe aus dem Inneren der Bandscheibe entnommen werden.

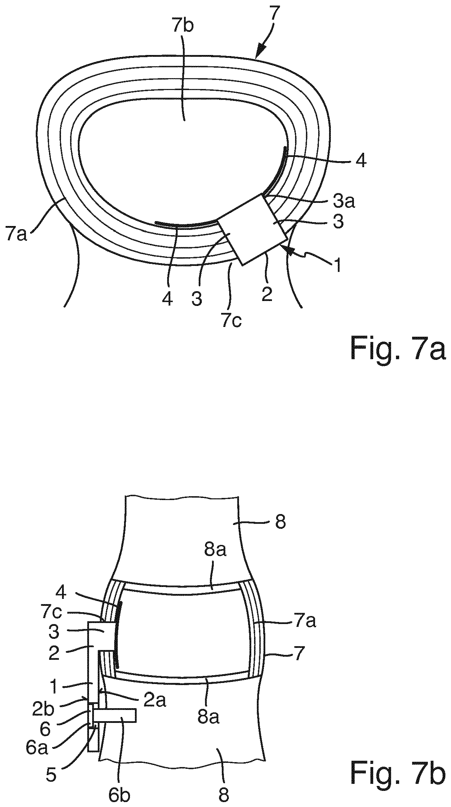

Die Figuren 7a und 7b deuten die Lage des in eine Bandscheibe eingebrachten Implantates in einem horizontalen und einem vertikalen Schnitt an. Das Lecksegel 4 liegt an der Innenseite des Anulus fibrosus 7a an und erstreckt sich deutlich über den Querschnitt des Defektes 7c hinaus. Der Halteabschnitt stützt das Lecksegel im Bereich des Defektes 7c ab, zumindest bis das Lecksegel eingeheilt bzw. eingewachsen ist. Der Halteabschnitt 3 und auch der Befestigungsschenkel 2 sowie gegebenenfalls auch das Fixierelement 6 können aus einem vom Körper eines Patienten resorbierbaren Material bestehen.

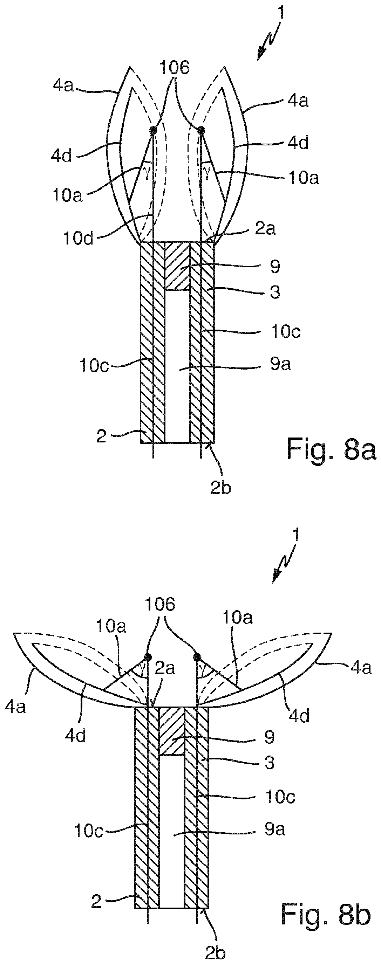

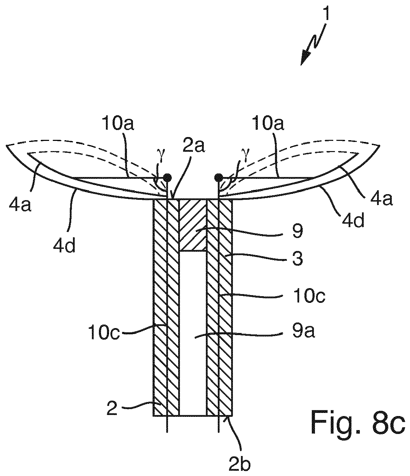

In Figur 8 a - c ist gemäß einer Ausführungsform der vorliegenden Erfindung ein Implantat 1 in einem Längsschnitt durch den Halteabschnitt 3 dargestellt. Das Implantat 1 weist für das Lecksegel 4 einen schirmartigen Aufspannmechanismus 10a, 10b und einen Zugdraht oder -Faden 10c auf, welcher sich durch den Halteabschnitt 3 und ein Ende des Befestigungsschenkels 2 hindurch erstreckt und mit zwei Segmenten 4a (nur angedeutet) des Lecksegels 4 derart verbunden ist, dass das Lecksegel 4 durch ein Ziehen an dem Draht oder Faden 10c von der Außenseite 2b her aufgespannt wird.

Jedes der Segmente 4a weist eine Verstärkung 4d auf, die in der Figur 8 nur angedeutet ist und die mit je einer Strebe 10a über eine Gelenkverbindung 10b mit einer Zugstange 10d verbunden ist, deren anderes Ende mit dem Zugfaden oder -draht 10c verbunden ist. Zur Verdeutlichung der Funktionsweise des Aufspannmechanismus ist das Lecksegel 4 in den Figuren 8a bis 8c in verschiedenen Öffnungspositionen dargestellt, wobei in Figur 8a das Lecksegel 4 zusammengefaltet bzw. geschlossen ist, in Figur 8b das Lecksegel 4 teilweise geöffnet ist und in Figur 8c das Lecksegel vollständig aufgespannt ist. Wenn der Zugfaden oder-draht während des Einbringens schon innerhalb des Defektes 7c unter Vorspannung gehalten wird, entfaltet sich das Lecksegel sobald es den Defekt 7c im Anulus fibrosus passiert hat.

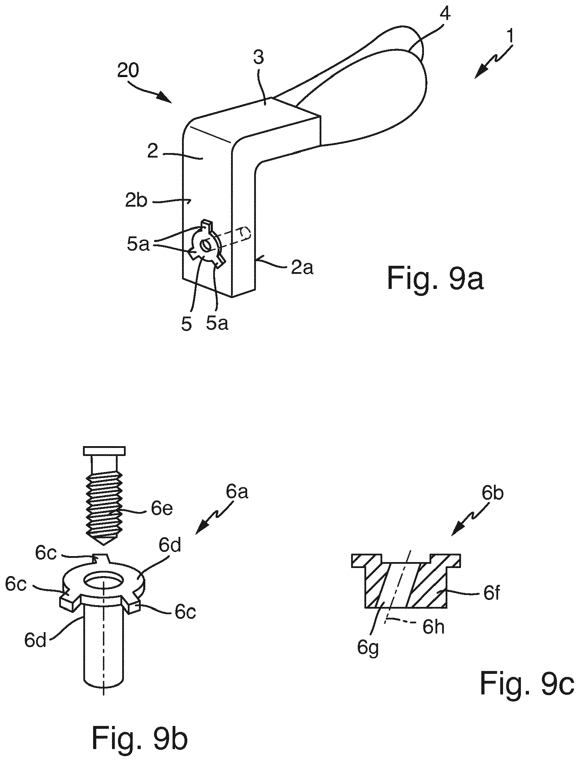

Die Figur 9 zeigt eine perspektivische Ansicht auf eine weitere Ausführungsform der vorliegenden Erfindung, bei der die Bohrung 5 im Befestigungsschenkel 2 Positioniermittel in Form von Randaussparungen 5a aufweist, die in 120° Abständen um eine zentrale Bohrung herum angeordnet sind. Diese Randaussparungen ermöglichen die Aufnahme einer passenden Hülse 6a, die korrespondierende radiale Fortsätze 6c aufweist und dienen der Verdrehsicherung der Hülsen 6a oder 6b. Zwei entsprechende, voneinander verschiedene Hülsen 6a, 6b sind in den Figuren 9b und 9c dargestellt.

Die Randaussparungen 5a und die radialen Fortsätze 6c ermöglichen die Anordnung der Hülsen 6a, 6b in der zentralen Bohrung 5 in drei verschiedenen Ausrichtungen, was insbesondere dann von Vorteil ist, wenn die Hülse ihrerseits eine Bohrung 6g aufweist, deren Achse geneigt zu der durch die Außenfläche 6f der Hülse 6a definierten Achse 6h verläuft. Die Zahl und Anordnung der Randaussparungen 5a kann selbstverständlich beliebig variiert werden um die Zahl der möglichen Orientierungen der Adapterhülse 6a oder 6b zu erhöhen oder zu verringern.

Damit können Fixierelemente, wie zum Beispiel Schrauben, je nach Ausrichtung der Hülse 6a und gegebenenfalls einer Durchgangsbohrung durch die Hülse aus drei verschiedenen Richtungen in den Wirbelkörper eingebracht werden, wobei der Chirurg die Möglichkeit hat, die Richtung nach dem geringstmöglichen Verletzungsgrad der auf dem Zugangsweg zu dem Defekt der Bandscheibe liegenden Knochen- und Weichteile auszuwählen. Alternativ können die Randaussparungen und Fortsätze als Verdrehsicherung dienen, wenn beispielsweise die in die Bohrung 5 einzusetzende Hülse einen Dübelansatz 6d aufweist, der sich in das Knochenmaterial des Wirbelkörpers 8 erstreckt. Auch ein nicht rotationssymmetrischer Schraubenkopf könnte mit einer zusätzlichen Hülse, die einerseits auf den Schraubenkopf aufgesteckt und gleichzeitig in die Bohrung 5 mit den Randaussparungen 5a eingerastet wird, gegen Verdrehung gesichert werden.

Beispiele geeigneter Hülsen 6a, 6b, wie sie in einigen Ausführungsformen der vorliegenden Erfindung zum Kombinieren mit einem erfindungsgemäßen Implantat 1 verwendet werden können, sind in den Figuren 9b und 9c dargestellt.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

### Bezugszeichenliste

- 1: Implantat

- 2: Befestigungsschenkel
- 2a: Innenseite des Befestigungsschenkels
- 2b: Außenseite des Befestigungsschenkels

- 3: Halteabschnitt
- 3a: Ende des Halteabschnitts

- 4: Lecksegel
- 4: Segment des Lecksegels
- 4b: Segmentspitze
- 4c: Längsachse des Segmentes
- 4d: Verstärkung

- 5: Bohrung zur Aufnahme eines Fixierelements
- 5a: Positioniermittel, Randaussparung

- 6a: Hülse
- 6b: Hülse
- 6c: Fortsätze
- 6d: Dübel, Dübelansatz
- 6e: Schraube, Gewindeschaft
- 6f: Außenwand der Adapterhülse
- 6g: Hülsenkanal
- 6h: Längsachse des Hülsenkanals

- 7: Bandscheibe
- 7a: Anulus fibrosus
- 7b: Nucleus pulposus
- 7c: Defekt

- 8: Wirbelkörper
- 8a: Endplatte

- 9: Applikationsport, Pfropfen
- 9a: Durchbrechung, Bohrung

- 5: Betätigungsmittel
- 10a: Strebe
- 10b: Gelenkverbindung
- 10c: Zugstange

## Patentansprüche

1. Implantat (1) zum Verschließen eines Defekts (7c) im Anulus fibrosus (7a) einer Bandscheibe (7) mit einem Halteabschnitt (3) zum Einführen in den Defekt (7c), einem mit einem inneren Ende des Halteabschnitts verbundenen flexiblen, Abdichtelement und einem mit dem äußeren Ende des Halteabschnitts verbundenen Befestigungselement (2), das an einem an die Bandscheibe angrenzenden Wirbelkörper (8) fixierbar ist, um den Halteabschnitt (3) in einer festen Position zu halten, wobei das flexible Abdichtelement innere Verstärkungen aufweist und in einer Draufsicht von vorn entlang der Längsachse des Halteabschnittes in einem ersten Zustand eine reduzierte Projektionsfläche hat und in einem zweiten Zustand eine erweiterte Projektionsfläche hat, die mindestens das Doppelte der reduzierten Projektionsfläche beträgt, **dadurch gekennzeichnet, dass** das Befestigungselement ein am äußeren Ende des Halteabschnitts angeordneter, zur Längsachse abgewinkelter Befestigungsschenkel (2) ist, der für eine seitliche Anlage an eine knöcherne Struktur eines zu der Bandscheibe benachbarten Wirbelkörpers ausgelegt und mit Hilfe eines gegenüber dem Befestigungsschenkel beweglichen Fixierelements (6) an dem Wirbelkörper befestigbar ist.

2. Implantat (1) nach Anspruch 1, **dadurch gekennzeichnet, dass** der Befestigungsschenkel im Abstand zu dem Halteabschnitt eine Querbohrung (5) für das Hindurchführen eines im Wirbelkörper zu verankernden Fixierelements (6) aufweist.

3. Implantat (1) nach Anspruch 2, **dadurch gekennzeichnet, dass** die Querbohrung (5) zur Längsrichtung des Halteabschnitts (3) unter einem Winkel im Bereich von 0° bis 30° geneigt ist.

4. Implantat (1) nach Anspruch 2 oder 3, **dadurch gekennzeichnet, dass** eine Adapterhülse (6f) vorgesehen ist, die in die Querbohrung (5) einführbar ist und die ihrerseits eine Bohrung aufweist, deren Achse zu der Längsachse der Querbohrung (5) unter einem Winkel im Bereich von 0° bis 30° geneigt ist.

5. Implantat (1) nach Anspruch 4, **dadurch gekennzeichnet, dass** die Adapterhülse (6f) in der Querbohrung (5) vormontierbar ist.

6. Implantat (1) nach einem der vorstehenden Ansprüche, **dadurch gekennzeichnet, dass** das Fixierelement (6) ein Nagel oder eine Schraube (6e), ein Dübel (6d) mit einer Schraube (6e) und vorzugsweise eine gesicherte Schraube (6e) mit einem Spreizdübel (6d) ist.

7. Implantat (1) nach einem der vorstehenden Ansprüche, **dadurch gekennzeichnet, dass** der Befestigungsschenkel und der Halteabschnitt einstückig ausgebildet sind, oder dass der Befestigungsschenkel und der Halteabschnitt lösbar und optional gelenkig miteinander verbunden sind.

8. Implantat (1) nach einem der vorstehenden Ansprüche, **dadurch gekennzeichnet, dass** das Abdichtelement am inneren Ende (3a) des Halteabschnitts (3) ein aufspannbares Lecksegel (4) ist, das in einer Draufsicht auf die Stirnseite des Halteabschnitts (3) in seinem aufgespannten Zustand allseitig über die Querschnittsfläche des Halteabschnitts (3) hinausragt und für das Verschließen des Defektes auf der Innenseite des Anulus fibrosus vorgesehen ist.

9. Implantat (1) nach Anspruch 8, **dadurch gekennzeichnet, dass** das Lecksegel (4) integrierte stab- oder drahtförmige Verstärkungen aufweist, die entweder eine Vorspannung in Richtung des aufgespannten Zustandes bereitstellen oder das Aufspannen des Lecksegels durch Manipulation der Verstärkungen von außen ermöglichen.

10. Implantat (1) nach Anspruch 8, **dadurch gekennzeichnet, dass** das Lecksegei (4) mindestens zwei, vorzugsweise mindestens drei Segmente (4a) aufweist, wobei benachbarte Segmente (4a) zumindest abschnittsweise miteinander verbunden sind und/oder sich in einem aufgespannten Zustand des Lecksegels (4) abschnittsweise überlappen.

11. Implantat (1) nach Anspruch 8 oder 9, **dadurch gekennzeichnet, dass** jedes Segment (4a) des Lecksegels (4) blattförmig ausgestaltet ist und eine sich bis zu der Blattspitze (4b) erstreckende Längsachse (4c) aufweist, wobei die Längsachsen (4c) zweier benachbarter Segmente (4a) in einem aufgespannten Zustand des Lecksegels (4) einen Winkel von 10° bis 120°, vorzugsweise von 45° bis 90° einschließen.

12. Implantat (1) nach einem der Ansprüche 8 bis 11, **dadurch gekennzeichnet, dass** durch den Halteabschnitt von seinem äußeren zu seinem Inneren Ende ein Betätigungsmittel (10) hindurchgeführt ist, das mit dem Lecksegel (4) verbunden ist und durch Betätigen das Aufspannen des Lecksegels (4) bewirkt.

13. Implantat (1) nach Anspruch 12, **dadurch gekennzeichnet, dass** das Betätigungsmittel (10) zusammen mit Verstärkungen des Lecksegels einen inversen Schirmmechanismus bildet.

14. Implantat (1) nach einem der Ansprüche 1 bis 13, **dadurch gekennzeichnet, dass** der Halteabschnitt (3) eine sich von seinem äußeren zum inneren Ende erstreckende Durchgangsbohrung aufweist, welche durch einen elastischen durchstoßbaren Pfropfen (11) verschlossen ist.

15. Implantat (1) nach Anspruch 14, **dadurch gekennzeichnet, dass** der Pfropfen (11) überwiegend aus Silikon besteht.

16. Implantat (1) nach Anspruch 14 oder 15, **dadurch gekennzeichnet, dass** der Pfropfen (11) in Richtung der Durchgangsbohrung des Halteabschnitts (3) eine Dicke von mindestens 2 mm, vorzugsweise mindestens 5 mm hat.

## Claims

1. Implant (1) for sealing a defect (7c) in the anulus fibrosus (7a) of an intervertebral disc (7), with a retaining portion (3) for insertion into the defect (7c), a flexible sealing element connected to an inner end of the retaining portion and a securing element (2) connected to the outer end of the retaining portion, which can be fixed to a vertebra (8) adjoining the intervertebral disc, in order to hold the retaining portion (3) in a fixed position, wherein the flexible sealing element has internal reinforcements and a reduced projected area in a top view from the front along the longitudinal axis of the retaining portion in a first state and has a widened projected area, which is at least twice the reduced projected area, in a second state, **characterized in that** the securing element is a securing limb (2) arranged on the outer end of the retaining portion and being angled relative to the longitudinal axis of the retaining portion, which securing limb is designed to be laid laterally against a bony structure of a vertebra neighbouring the intervertebral disc and can be secured to the vertebra with the aid of a fixing element (6) which is movable relative to the securing limb.

2. Implant (1) according to claim 1, **characterized in that**, at a distance from the retaining portion, the securing limb has a cross hole (5) for a fixing element (6), to be anchored in the vertebra, to be guided through.

3. Implant (1) according to claim 2, **characterized in that** the cross hole (5) is inclined relative to the longitudinal direction of the retaining portion (3) at an angle in the range of from 0° to 30°.

4. Implant (1) according to claim 2 or 3, **characterized in that** an adapter sleeve (6f) is provided which can be inserted into the cross hole (5) and which itself has a hole, the axis of which is inclined relative to the longitudinal axis of the cross hole (5) at an angle in the range of from 0° to 30°.

5. Implant (1) according to claim 4, **characterized in that** the adapter sleeve (6f) is pre-installable in the cross hole (5).

6. Implant (1) according to one of the preceding claims, **characterized in that** the fixing element (6) is a nail or a screw (6e), a screw anchor (6d) with a screw (6e) and preferably a secured screw (6e) with an expansion anchor (6d).

7. Implant (1) according to one of the preceding claims, **characterized in that** the securing limb and the retaining portion are formed in one piece, or **in that** the securing limb and the retaining portion are connected to each other in a detachable and optionally articulated manner.

8. Implant (1) according to one of the preceding claims, **characterized in that**, on the inner end (3a) of the retaining portion (3), the sealing element is a fothering (4) that can be spread out which projects beyond the cross-sectional area of the retaining portion (3) on all sides in a top view onto the end face of the retaining portion (3) in its spread-out state and is provided to seal the defect on the inner side of the anulus fibrosus.

9. Implant (1) according to claim 8, **characterized in that** the fothering (4) has integrated rod- or wire-shaped reinforcements, which either provide a pretension in the direction of the spread-out state or make it possible to spread out the fothering by manipulation of the reinforcements from outside.

10. Implant (1) according to claim 8, **characterized in that** the fothering (4) has at least two, preferably at least three segments (4a), wherein neighbouring segments (4a) are connected to each other at least in portions and/or overlap in portions in a spread-out state of the fothering (4).

11. Implant (1) according to claim 8 or 9, **characterized in that** each segment (4a) of the fothering (4) is designed leaf-shaped and has a longitudinal axis (4c) extending up to the leaf tip (4b), wherein the longitudinal axes (4c) of two neighbouring segments (4a) form an angle of from 10° to 120°, preferably of from 45° to 90°, in a spread-out state of the fothering (4).

12. Implant (1) according to one of claims 8 to 11, **characterized in that** an actuation means (10), which is connected to the fothering (4) and brings about the spreading-out of the fothering (4) by actuation, is guided through the retaining portion from its outer to its inner end.

13. Implant (1) according to claim 12, **characterized in that** the actuation means (10), together with reinforcements of the fothering, forms an inverse umbrella mechanism.

14. Implant (1) according to one of claims 1 to 13, **characterized in that** the retaining portion (3) has a through hole, extending from its outer to the inner end, which is sealed by an elastic, pierceable plug (11).

15. Implant (1) according to claim 14, **characterized in that** the plug (11) consists predominantly of silicone.

16. Implant (1) according to claim 14 or 15, **characterized in that** the plug (11) has a thickness of at least 2 mm, preferably at least 5 mm, in the direction of the through hole of the retaining portion (3).

## Revendications

1. Implant (1) pour l'obturation d'un défaut (7c) dans l'anneau fibreux (7a) d'un disque intervertébral (7), avec une partie de maintien (3) pour l'introduction dans le défaut (7c), un élément d'étanchéité flexible attaché à une extrémité intérieure de la partie de maintien et un élément de fixation (2) attaché à une extrémité extérieure de la partie de maintien, qui est adapté pour être fixé sur un corps de vertèbre (8) adjacent au disque intervertébral pour maintenir la partie de maintien (3) dans une position fixe, l'élément d'étanchéité flexible comprenant des renforcements intérieurs et présentant, en une vue de dessus par devant, le long de l'axe longitudinal de la partie de maintien, une surface de projection réduite dans un premier état et une surface de projection élargie qui est au moins le double de la surface de projection réduite, dans un deuxième état, **caractérisé en ce que** l'élément de fixation est une branche de fixation (2) disposée à l'extrémité extérieure de la partie de maintien et formant un angle avec l'axe longitudinal, qui est configurée pour un appui latéral sur une structure osseuse d'un corps de vertèbre avoisinant au disque intervertébral et qui est adaptée pour pouvoir être fixée au corps de vertèbre à l'aide d'un élément de fixation (6) mobile par rapport à la branche de fixation.

2. Implant (1) selon la revendication 1, **caractérisé en ce que** la branche de fixation comprend un perçage transversal (5) destiné au passage d'un élément de fixation (6) qui doit être ancré dans le corps de vertèbre.

3. Implant (1) selon la revendication 2, **caractérisé en ce que** le perçage transversal (5) est incliné, par rapport à la direction longitudinale de la partie de maintien (3), d'un angle dans la plage de 0° à 30°.

4. Implant (1) selon la revendication 2 ou 3, **caractérisé en ce qu'**il est prévu un manchon d'adaptation (6f) qui est susceptible d'être introduit dans le perçage transversal (5) et qui, elle-même, comprend un perçage dont l'axe est incliné, par rapport à l'axe longitudinal du perçage transversal (5), d'un angle dans la plage de 0° à 30°.

5. Implant (1) selon la revendication 4, **caractérisé en ce que** le manchon d'adaptation (6f) est susceptible d'être prémonté dans le perçage transversal (5).

6. Implant (1) selon l'une des revendications précédentes, **caractérisé en ce que** l'élément de fixation (6) est un clou ou une vis (6e), une cheville (6d) avec une vis (6e) et, de préférence, une vis sécurisée (6e) avec une cheville expansible (6d).

7. Implant (1) selon l'une des revendications précédentes, **caractérisé en ce que** la branche de fixation et la partie de maintien sont formées en une seule pièce ou **en ce que** la branche de fixation et la partie de maintien sont attachées l'une à l'autre de manière amovible ou, en option, de manière articulée.

8. Implant (1) selon l'une des revendications précédentes, **caractérisé en ce que** l'élément d'étanchéité à l'extrémité intérieure (3a) de la partie de maintien (3) est un voile de fuite (4) qui, en une vue de dessus sur la face frontale de la partie de maintien (3), dans son état déployé, dépasse de tous le côtés la surface de section transversale de la partie de maintien (3) et est prévu pour l'obturation du défaut sur la face intérieure de l'anneau fibreux.

9. Implant (1) selon la revendication 8, **caractérisé en ce que** le voile de fuite (4) comprend des renforcements intégrés sous la forme de tiges ou de fils métalliques qui mettent à disposition une précontrainte dans la direction de l'état déployé ou qui permettent de déployer le voile de fuite de l'extérieur par manipulation des renforcements.

10. Implant (1) selon la revendication 8, **caractérisé en ce que** le voile de fuite (4) comprend au moins deux, de préférence au moins trois segments (4a), des segments (4a) avoisinants étant attachés les uns aux autres au moins par tronçon et/ou se chevauchant par tronçon dans un état déployé du voile de fuite (4).

11. Implant (1) selon la revendication 8 ou 9, **caractérisé en ce que** chaque segment (4a) du voile de fuite (4) est configuré en forme de feuille et comprend un axe longitudinal (4c) s'étendant jusqu'à la pointe de feuille (4b), les axes longitudinaux (4c) de deux segments (4a) avoisinants, en un état déployé du voile de fuite (4), enfermant un angle de 10° à 120°, de préférence de 45° à 90°.

12. Implant (1) selon l'une des revendications 8 à 11, **caractérisé en ce qu'**un moyen d'actionnement (10) est passé par la partie de maintien de l'extrémité extérieure à l'extrémité intérieure de cette dernière, ledit moyen étant attaché au voile de fuite (4) et causant, par son actionnement, le déploiement du voile de fuite (4).

13. Implant (1) selon la revendication 12, **caractérisé en ce que** le moyen d'actionnement (10) forme, ensemble avec des renforcements du voile de fuite, un mécanisme inverse de parapluie.

14. Implant (1) selon l'une des revendications 1 à 13, **caractérisé en ce que** la partie de maintien (3) comprend un perçage de passage s'étendant de son extrémité extérieure à son extrémité intérieure, qui est obturé par un bouchon élastique (11) adapté pour être perforé.

15. Implant (1) selon la revendication 14, **caractérisé en ce que** le bouchon (11) consiste principalement en silicone.

16. Implant (1) selon la revendication 14 ou 15, **caractérisé en ce que** le bouchon (11) présente, dans la direction du perçage de passage de la partie de maintien (3), une épaisseur d'au moins 2 mm, de préférence d'au moins 5 mm.
